# EUROPEAN PATENT SPECIFICATION

(11) **EP 2 384 623 B1**
(45) Date of publication and mention of the grant of the patent: **21.11.2012**
(21) Application number: 11165106.3
(22) Date of filing: 06.05.2011
(51) Int. Cl.: A01N 25/14, A01N 25/24, A01N 43/66, A01N 59/00, A01N 59/20, A61K 9/14, A61K 31/145, A61K 31/197, A61K 31/53, A61K 31/723, A61K 33/34, A61K 47/00, A61K 36/481, A61K 36/48, A61P 31/02

(54) **Powder composition for the prevention and treatment of animal hoof diseases**
Pulverzusammensetzung zur Behandlung von Huferkrankung
Composition pulvérulente pour le traitement des maladies du sabot

(30) Priority: 06.05.2010 IT MI20100807
(43) Date of publication of application: 09.11.2011
(73) Proprietor: I.C.F. S.r.l., 26020 Palazzo Pignano (CR) (IT)
(72) Inventor: Gelmi, Fabio, 26100, CREMONA (IT); Venturini, Maurizio, 26100, CREMONA (IT)
(74) Representative: Gervasi, Gemma

(56) References cited:
- EP-A2- 1 932 425
- WO-A1-02/102352
- WO-A2-2007/070655
- US-A1- 2007 295 517

## Description

### FIELD OF THE INVENTION

The present invention relates to a powder composition for the prevention and treatment of animal foot diseases and uses thereof.

In particular, the present invention relates to a composition for preventing animal hoof infections and for treating inflammatory states of the hoof.

### STATE OF THE ART

In the state of the art, the treatment of animal foot diseases, such as the treatment of abscesses (white line disease), the treatment of laminitis which involves oedema, haemorrhage and necrosis of the corium tissue, as well as the eradication of *Bacteroides nodosus,* the cause of irritating dermatitis which deteriorates into ulcers, is based on local therapies which employ products based on quaternary ammonium salts, peracetic acid, formalin, paraformaldehyde, copper sulphate and organic chlorine-based compounds. The organic chlorine-based compounds perform wide spectrum antibacterial action and are used in the preparation of many products, whose proven bacteriostatic, bactericidal, antifungal and antiviral action is used along with their capacity to prevent and slow down foot diseases.

EP 1932425 relates to a powder composition for disinfecting the teats of dairy animals containing chloramine-T, EDTA, real aloe extract and xanthan rubber.

Organic chlorine-based products are also used in preparations intended for human use such as skin disinfectants. In the veterinary field, the use of organic chlorine-based compositions, known in the state of the art for treating animal foot diseases, has various drawbacks mainly ascribable to high dosages needed to obtain significant beneficial effects.

A first drawback associated with the use of organic chlorine compounds at high dosages derives, for example, from the irritating and inflammatory effect on the animals' hooves and skin; moreover, if inhaled it is irritating to the animals' airways. The onset of said side effects can force treatment to be suspended before the desired results have been achieved.

A second drawback associated with the use of the known organic chlorine-based compositions, is associated with the fact that a high dosage thereof can also lead to irritating and inflammatory effects on the user during product use.

In the veterinary field, peracetic acid-based compounds, known in the state of the art for treating animal foot diseases, present the drawback of releasing acetic acid which has an irritant effect. Also, preparations of this type are not very stable. Again in the veterinary field, formalin-based products, known in the art for treating animal foot diseases, present the drawback of containing an active principle with mutagenic characteristics. Again in the veterinary field, copper sulphate-based products, known in the state of the art for treating animal foot diseases (see WO 02/102352, WO 2007/070655 and US 2007/0295517), present the drawback that at high dosages, copper sulphate is difficult to dispose of as being poorly bio-degradable and leading to a persistent build-up in the soil.

The object of the present invention is to provide a product that is effective for the applications indicated above while at the same time enabling the relevant drawbacks of the known treatments to be overcome.

### SUMMARY OF THE INVENTION

The above indicated object has been achieved by the use of a powder composition comprising:
a) from 10 to 40% by weight on the total composition weight, of at least one chlorinated organic compound selected from the group consisting of chlorinated isocyanuric acid or its salts, aromatic chloro-sulphonamide or its salts, and mixtures thereof,
b) from 0 to 30% by weight on the total composition weight, of copper sulphate,
c) from 1 to 20% by weight on the total composition weight, of EDTA or its salt,
d) from 30 to 75% by weight on the total composition weight, of a natural gum,
e) from 0 to 6% by weight of an inorganic carbonate, and veterinarily acceptable adjuvants and/or excipients,
as an anti-inflammatory and/or antimicrobial agent for the prevention and treatment of foot diseases in ungulates.

In another aspect, the present invention concerns a powder composition comprising:
a) from 10 to 40% by weight on the total composition weight, of at least one chlorinated organic compound selected from the group consisting of chlorinated isocyanuric acid or its salts, aromatic chloro-sulphonamide or its salts, and mixtures thereof,
b) from 3 to 20% by weight on the total composition weight, of copper sulphate,
c) from 1 to 20% by weight on the total composition weight, of EDTA or its salt,
d) from 30 to 75% by weight on the total composition weight, of a natural gum,
e) from 0 to 6% by weight of an inorganic carbonate, and veterinarily acceptable adjuvants and/or excipients.

The characteristics and advantages of the present invention will be evident from the detailed description given below, and from the illustrative non-limiting working examples.

### DETAILED DESCRIPTION OF THE INVENTION

The invention therefore relates to the use of a powder composition comprising:
a) from 10 to 40% by weight on the total composition weight, of at least one chlorinated organic compound selected from the group consisting of chlorinated isocyanuric acid or its salts, aromatic chloro-sulphonamide or its salts, and mixtures thereof,
b) from 0 to 30% by weight on the total composition weight, of copper sulphate,
c) from 1 to 20% by weight on the total composition weight, of EDTA or its salt,
d) from 30 to 75% by weight on the total composition weight, of a natural gum,
e) from 0 to 6% by weight of an inorganic carbonate, and veterinarily acceptable adjuvants and/or excipients,
as an anti-inflammatory and/or antimicrobial agent for the prevention and treatment of foot diseases in ungulates.

The invention relates to the use of the powder composition of the invention in the veterinary field, as it has enabled significantly satisfying results to be attained in treating animal foot diseases, as well as in preventing animal hoof infections and in treating inflammatory states of the hoof.

Therefore, the powder composition of the invention finds advantageous and convenient application as an anti-inflammatory and antimicrobial agent for treating foot diseases in ungulates.

Moreover, the powder composition of the invention finds advantageous and convenient usage in the prevention of hoof diseases in animals.

It has been surprisingly found that said powder composition is able to effectively treat foot diseases in ungulates by using low dosages of a chlorinated organic compound, thereby completely eliminating the use of formalin, quaternary ammonium salts, peracetic acid or paraformaldehyde. The efficacy at low dosages is made possible by the particular formulation which enables an increase in the contact time of the active principle, due to the presence in the composition of a natural gum powder. In this respect, since said composition is mixed with an aqueous medium prior to use, the natural gum powder generates a high viscosity colloidal dispersion able to adhere to foot extremities of animals so as to prolong contact time of the active principles with the areas to be treated.

In said uses, the composition is firstly dispersed in an aqueous medium, preferably water, to form a high viscosity gel prior to application.

Said uses relate to the treatment of foot diseases in all species of hoofed animals, preferably cattle, buffalo, horses, sheep, goats and pigs.

A further advantage of the use of a powder composition is the high stability of chlorinated organic compounds compared to that of the same compounds but in aqueous solution. This means that the shelf life of this composition is greatly and significantly increased, not only with respect to the corresponding aqueous solutions, but also with respect to known products.

Hence, it has surprisingly been found that by combining a natural gum, a chlorinated organic compound, and EDTA in suitable weight percentages, a powder composition for veterinary use can be obtained having a high antimicrobial and a simultaneous anti-inflammatory efficacy, advantageously at low dosages.

In particular, said composition is advantageously used for foot diseases including white line disease, digital dermatitis (Mortellaro Disease), interdigital dermatitis, solar ulcers, white line diathesis, laminitis, sole abscesses and interdigital phlegmon.

In a preferred embodiment, said chlorinated organic compound is selected from the group consisting of trichloroisocyanuric acid, sodium trichloroisocyanurate, sodium dichloroisocyanurate, Chloramine T and mixtures thereof.

Preferably, the powder composition of the invention comprises from 15 to 45% by weight of said at least one organic chlorine compound or a derivative thereof, more preferably from 25 to 40% by weight.

Preferably, in said powder composition, EDTA or its salt is present, wherein said salt is preferably chosen from EDTA disodium salt and EDTA tetrasodium salt. In a preferred embodiment, the powder composition comprises tetrasodium salt (Na₄EDTA).

Preferably, the composition of the invention comprises from 4 to 15% by weight of said EDTA or its salt, more preferably from 4 to 9% by weight.

Preferably, the powder composition comprises from 35 to 70% by weight of natural gum, preferably from 40 to 60% by weight. Said natural gum is preferably selected from the group consisting of gum Arabic, tragacanth, guar gum, and xanthan gum.

In a preferred embodiment, said natural gum is xanthan gum.

It has been surprisingly found that the presence of natural gum generates a gel on mixing the powder with an aqueous medium. The gel consistency of the composition of the invention increases the contact time, as previously stated, thereby enhancing the action of organic chlorine compounds and their derivatives, copper sulphate and EDTA, hence enabling these compounds, for the same treatment efficacy, to be used in advantageously and significantly lower amounts than concentrations known in the state of the art and used for the same veterinary purposes.

The composition can also comprise inorganic carbonate, which is chosen from ammonium carbonate, barium carbonate, cadmium carbonate, calcium carbonate, caesium carbonate, lithium carbonate, magnesium carbonate, manganese carbonate, lead carbonate, potassium carbonate, sodium carbonate, strontium carbonate and mixtures thereof.

Preferably, the powder composition further comprises a powder dyeing agent. After mixing the composition with an aqueous medium prior to use, said dyeing agent advantageously confers an intense colour thereto, thus enabling the treated areas to be marked. More preferably, said dyeing agent presents an intense blue colour when in water. In the composition of the invention, the dyeing agent is present in an amount from 0.01 to 20% by weight on the total composition weight, preferably from 0.1 to 10%, more preferably from 1 to 5%.

The dosage in the use of the composition of the present invention is dependent on the foot disease which is to be prevented or treated. Preferably, the powder composition is dispersed in foot baths at a dilution such that the active chlorine concentration is at least 250 ppm, preferably at least 1000 ppm. The treatment is then preferably carried out by setting a contact time of 5 minutes once a week for the first month followed by a contact time of 5 minutes every 15 days.

In a preferred embodiment, the powder composition for the previously described use of the invention consists of:
- 25 to 35% by weight of trichloroisocyanuric acid or its salt in powder form,
- 5 to 15% by weight of EDTA sodium salt,
- 45 to 60% by weight of xanthan gum,
- 0 to 5% by weight of sodium or potassium carbonate, and
- 2 to 5% by weight of dyeing agent.

In a particularly preferred embodiment, the powder composition for the previously described use of the invention consists of:
- 27 to 30% by weight of trichloroisocyanuric acid or its salt in powder form,
- 8 to 10% by weight of EDTA sodium salt,
- 53 to 57% by weight of xanthan gum,
- 2 to 4% by weight of sodium or potassium carbonate, and
- 2 to 4% by weight of dyeing agent.

In another aspect, the present invention concerns a powder composition comprising:
a) from 10 to 40% by weight, on the total composition weight, of at least one chlorinated organic compound selected from the group consisting of chlorinated isocyanuric acid or its salts, aromatic chloro-sulphonamide or its salts, and mixtures thereof,
b) from 3 to 20% by weight on the total composition weight, of copper sulphate,
c) from 1 to 20% by weight on the total composition weight, of EDTA or its salt,
d) from 30 to 75% by weight on the total composition weight, of a natural gum,
e) from 0 to 6% by weight of an inorganic carbonate, and veterinarily acceptable adjuvants and/or excipients.

It should be understood that all aspects identified as preferred and advantageous for the previously described use, are accordingly considered to be preferred and advantageous for this powder composition.

The powder composition comprises 10 to 20% by weight of copper sulphate. It has been surprisingly found that copper sulphate advantageously stabilizes hoof hardness, thus preventing excess moisture from softening the horn which would typically lead to the development of germs and infections.

Also the above-described powder composition comprising copper sulphate finds advantageous use as an anti-inflammatory and/or antimicrobial agent for the prevention and treatment of foot diseases in ungulates.

The powder compositions of the present invention are prepared by mixing powder compounds in the aforedefined weight ratios.

Working examples of the present invention provided for illustrative purposes are reported herein below.

### Example 1

In accordance with the present invention, 100 g of a composition for veterinary use in powder form were prepared by mixing:
- 50.63 g of xanthan gum,
- 29.77 g of trichloroisocyanuric acid powder,
- 11.58 g of copper sulphate,
- 4.86 g of EDTA tetrasodium salt,
- 3.16 g of dyeing agent.

The composition was used in the treatment of a bacterial foot ulcer in an adult cow once a week for a month, by applying the product to coat the entire hoof.

The use of the composition in accordance with the present invention has allowed the animal to be healed of its bacterial ulcer without the appearance of those side effects typically found in therapies with compositions based on common active principles used in the state of the art for foot treatments.

Furthermore, healthy cattle regularly treated with the same composition did not develop said disease, demonstrating that the composition of the invention also provides an effective prophylactic activity.

### Example 2

In accordance with the present invention, 12.5 kg of a composition for veterinary use in powder form were prepared by mixing:
- 6.988 kg of xanthan gum,
- 3.5 kg of trichloroisocyanuric acid powder,
- 1.1175 kg of EDTA,
- 0.395 kg of ferric ammonium ferrocyanide dyeing agent (Unipure Blue LC685), and
- 0.50 kg of sodium carbonate.

The composition was used in the treatment of white line disease in an adult cow once a week for a month, by applying the product to coat the entire hoof.

The use of the composition in accordance with the present invention has enabled the animal to be healed of white line disease without the appearance of those side effects typically found in therapies with compositions based on common active principles used in the state of the art for foot treatments.

Furthermore, healthy cattle regularly treated with the same composition did not develop said disease, demonstrating that the composition of the invention also provides an effective prophylactic activity.

### Example 3

In accordance with the present invention, 2.5 kg of a composition for veterinary use in powder form were prepared by mixing:
- 1.26575 kg of xanthan gum,
- 0.74425 kg of trichloroisocyanuric acid powder,
- 0.411 kg of EDTA,
- 0.079 kg of ferric ammonium ferrocyanide dyeing agent (Unipure Blue LC685). The composition was used in the treatment of digital dermatitis in an adult sheep once a week for a month, by applying the product to coat the entire hoof.

The use of the composition of the present invention has enabled the animal to be healed of digital dermatitis without the appearance of those side effects typically found in therapies with compositions based on common active principles used in the state of the art for foot treatments.

Furthermore, healthy sheep regularly treated with the same composition did not develop said disease, demonstrating that the composition of the invention also provides an effective prophylactic activity.

## Claims

1. Powder composition comprising:
a) from 10 to 40% by weight, on the total composition weight, of at least one chlorinated organic compound selected from the group consisting of chlorinated isocyanuric acid or its salts, aromatic chloro-sulphonamide or its salts, and mixtures thereof,
b) from 0 to 30% by weight on the total composition weight, of copper sulphate,
c) from 1 to 20% by weight on the total composition weight, of EDTA or its salt,
d) from 30 to 75% by weight on the total composition weight, of a natural gum,
e) from 0 to 6% by weight of an inorganic carbonate, and
veterinarily acceptable adjuvants and/or excipients,
as an anti-inflammatory and/or antimicrobial agent for use in the prevention and treatment of foot diseases in ungulates.

2. The composition for use of claim 1, wherein said foot diseases are white line disease, digital dermatitis (Mortellaro Disease), interdigital dermatitis, solar ulcers, white line diathesis, laminitis, sole abscesses and interdigital phlegmon.

3. The composition for use of claim 1 or 2, wherein said powder composition further comprises a powder dyeing agent.

4. The composition for use of any one of claims 1-3, wherein said powder composition is dissolved in an aqueous medium to form a gel, prior to application.

5. The composition for use of claim 4, wherein said powder composition is dissolved in an aqueous medium until a concentration of at least 250 ppm of chlorine is obtained.

6. The composition for use of any one of claims 1-5, wherein said powder composition consists of:
- 25 to 35% by weight of sodium trichloroisocyanurate,
- 5 to 15% by weight of EDTA sodium salt,
- 45 to 60% by weight of xanthan gum,
- 0 to 5% by weight of sodium or potassium carbonate, and
- 2 to 5% by weight of dyeing agent.

7. The composition for use of claim 6, wherein said powder composition consists of:
- 27 to 30% by weight of sodium trichloroisocyanurate,
- 8 to 10% by weight of EDTA sodium salt,
- 53 to 57% by weight of xanthan gum,
- 2 to 4% by weight of sodium or potassium carbonate, and
- 2 to 4% by weight of dyeing agent.

8. A powder composition comprising:
a) from 10 to 40% by weight, on the total composition weight, of at least one chlorinated organic compound selected from the group consisting of chlorinated isocyanuric acid or its salts, aromatic chloro-sulphonamide or its salts, and mixtures thereof,
b) from 3 to 20% by weight on the total composition weight, of copper sulphate,
c) from 1 to 20% by weight on the total composition weight, of EDTA or its salt,
d) from 30 to 75% by weight on the total composition weight, of a natural gum,
e) from 0 to 6% by weight of an inorganic carbonate, and
veterinarily acceptable adjuvants and/or excipients.

9. The composition of claim 8, wherein said chlorinated organic compound is selected from the group consisting of sodium trichloroisocyanurate, sodium dichloroisocyanurate, Chloramine T and mixtures thereof.

10. The composition of claim 8 or 9, comprising from 25 to 40% by weight of said at least one chlorinated organic compound.

11. The composition of any one of claims 8-10, comprising from 4 to 15% by weight of said EDTA or its salt, preferably from 4 to 9% by weight.

12. The composition of any one of claims 8-11, comprising from 35 to 70% by weight of said natural gum, preferably from 40 to 60% by weight.

13. The composition of any one of claims 8-12, wherein said natural gum is selected from the group consisting of gum Arabic, tragacanth, guar gum, and xanthan gum.

14. The composition of any one of claims 8-13, wherein said inorganic carbonate is ammonium carbonate, barium carbonate, cadmium carbonate, calcium carbonate, caesium carbonate, lithium carbonate, magnesium carbonate, manganese carbonate, lead carbonate, potassium carbonate, sodium carbonate, strontium carbonate and mixtures thereof.

15. The composition of any one of claims 8-14, further comprising a powder dyeing agent.

## Patentansprüche

1. Pulverzusammensetzung, die Folgendes umfasst:
a) mindestens eine organische Chlorverbindung, ausgewählt aus der Gruppe bestehend aus chlorierter Isocyanursäure oder ihren Salzen, aromatischem Chlorsulfonamid oder seinen Salzen und Mischungen davon, in einer Menge von 10 bis 40 Gew.-% in Bezug auf das Gesamtgewicht der Zusammensetzung,
b) Kupfersulfat in einer Menge von 0 bis 30 Gew.-% in Bezug auf das Gesamtgewicht der Zusammensetzung,
c) EDTA oder ein Salz davon in einer Menge von 1 bis 20 Gew.-% in Bezug auf das Gesamtgewicht der Zusammensetzung,
d) einen natürlichen Gummi in einer Menge von 30 bis 75 Gew.-% in Bezug auf das Gesamtgewicht der Zusammensetzung,
e) ein anorganisches Carbonat in einer Menge von 0 bis 6 Gew.-%, und
tierärztlich unbedenkliche Hilfsstoffe und/oder Grundstoffe,
als entzündungshemmendes Mittel und/oder antimikrobielles Mittel für die Verwendung in der Vorbeugung und Behandlung von Fußkrankheiten bei Ungulata.

2. Zusammensetzung für die Verwendung nach Anspruch 1, wobei es sich bei den Fußkrankheiten um Folgendes handelt: White Line Disease, Dermatitis digitalis (Mortellaro'sche Krankheit), Zwischenklauenentzündung, Klauensohlengeschwüre, White-Line-Diathese, Laminitis, Sohlenabszesse und Zwischenklauenphlegmone.

3. Zusammensetzung für die Verwendung nach Anspruch 1 oder 2, wobei die Pulverzusammensetzung weiterhin ein Farbstoffpulver umfasst.

4. Zusammensetzung für die Verwendung nach einem der Ansprüche 1-3, wobei die Pulverzusammensetzung vor der Applikation in einem wässrigen Medium unter Bildung eines Gels gelöst wird.

5. Zusammensetzung für die Verwendung nach Anspruch 4, wobei die Pulverzusammensetzung in einem wässrigen Medium gelöst wird bis man zu einer Konzentration von mindestens 250 ppm Chlor gelangt.

6. Zusammensetzung für die Verwendung nach einem der Ansprüche 1-5, wobei die Pulverzusammensetzung aus Folgendem besteht
- 25 bis 35 Gew.-% Natriumtrichlorisocyanurat,
- 5 bis 15 Gew.-% EDTA-Natriumsalz,
- 45 bis 60 Gew.-% Xanthangummi,
- 0 bis 5 Gew.-% Natrium- oder Kaliumcarbonat und
- 2 bis 5 Gew.-% Farbstoff.

7. Zusammensetzung für die Verwendung nach Anspruch 6, wobei die Pulverzusammensetzung aus Folgendem besteht:
- 27 bis 30 Gew.-% Natriumtrichlorisocyanurat,
- 8 bis 10 Gew.-% EDTA-Natriumsalz,
- 53 bis 57 Gew.-% Xanthangummi,
- 2 bis 4 Gew.-% Natrium- oder Kaliumcarbonat und
- 2 bis 4 Gew.-% Farbstoff.

8. Pulverzusammensetzung, die Folgendes umfasst:
a) mindestens eine organische Chlorverbindung, ausgewählt aus der Gruppe bestehend aus chlorierter Isocyanursäure oder ihren Salzen, aromatischem Chlorsulfonamid oder seinen Salzen und Mischungen davon, in einer Menge von 10 bis 40 Gew.-% in Bezug auf das Gesamtgewicht der Zusammensetzung,
b) Kupfersulfat in einer Menge von 3 bis 20 Gew.-% in Bezug auf das Gesamtgewicht der Zusammensetzung,
c) EDTA oder ein Salz davon in einer Menge von 1 bis 20 Gew.-% in Bezug auf das Gesamtgewicht der Zusammensetzung,
d) einen natürlichen Gummi in einer Menge von 30 bis 75 Gew.-% in Bezug auf das Gesamtgewicht der Zusammensetzung,
e) ein anorganisches Carbonat in einer Menge von 0 bis 6 Gew.-%, und
tierärztlich unbedenkliche Hilfsstoffe und/oder Grundstoffe.

9. Zusammensetzung nach Anspruch 8, wobei die organische Chlorverbindung aus der Gruppe bestehend aus Natriumtrichlorisocyanurat, Natriumdichlorisocyanurat, Chloramin T und Mischungen davon ausgewählt ist.

10. Zusammensetzung nach Anspruch 8 oder 9, die 25 bis 40 Gew.-% dieser mindestens einen organischen Chlorverbindung umfasst.

11. Zusammensetzung nach einem der Ansprüche 8-10, die 4 bis 15 Gew.-%, vorzugweise 4 bis 9 Gew.-%, der EDTA oder eines Salzes davon umfasst.

12. Zusammensetzung nach einem der Ansprüche 8-11, die 35 bis 70 Gew.-%, vorzugweise 40 bis 60 Gew.-%, des natürlichen Gummis umfasst.

13. Zusammensetzung nach einem der Ansprüche 8-12, wobei der natürliche Gummi aus der Gruppe bestehend aus Gummi arabicum, Traganth, Guargummi und Xanthangummi ausgewählt ist.

14. Zusammensetzung nach einem der Ansprüche 8-13, wobei es sich bei dem anorganischen Carbonat um Ammoniumcarbonat, Bariumcarbonat, Cadmiumcarbonat, Kalziumcarbonat, Caesiumcarbonat, Lithiumcarbonat, Magnesiumcarbonat, Mangancarbonat, Bleicarbonat, Kaliumcarbonat, Natriumcarbonat, Strontiumcarbonat und Mischungen davon handelt.

15. Zusammensetzung nach einem der Ansprüche 8-14, die weiterhin ein Farbstoffpulver umfasst.

## Revendications

1. Composition de poudre comprenant :
a) de 10 à 40 % en poids, par rapport au poids total de la composition, d'au moins un composé organique chloré choisi dans le groupe consistant en l'acide isocyanurique chloré ou ses sels, un chlorosulfonamide aromatique ou ses sels, et leurs mélanges,
b) de 0 à 30 % en poids, par rapport au poids total de la composition, de sulfate de cuivre,
c) de 1 à 20 % en poids, par rapport au poids total de la composition, d'EDTA ou de son sel ;
d) de 30 à 75 % en poids, par rapport au poids total de la composition, d'une gomme naturelle,
e) de 0 à 6 % en poids d'un carbonate inorganique,
et
des adjuvants et/ou excipients acceptables sur le plan vétérinaire,
en tant qu'agent anti-inflammatoire et/ou antimicrobien à utiliser dans la prévention et le traitement de maladies des pieds chez les ongulés.

2. Composition à utiliser selon la revendication 1, dans laquelle lesdites maladies des pieds sont une maladie de la ligne blanche, une dermatite digitée (maladie de Mortellaro), une dermatite interdigitée, des ulcères solaires, une diathèse de la ligne blanche, une laminite, des abcès de la sole et un phlegmon interdigité.

3. Composition à utiliser selon la revendication 1 ou 2, dans laquelle ladite composition de poudre comprend en outre un agent de teinture de poudre.

4. Composition à utiliser selon l'une quelconque des revendications 1 à 3, dans laquelle ladite composition de poudre est dissoute dans un milieu aqueux pour former un gel, avant application.

5. Composition à utiliser selon la revendication 4, dans laquelle ladite composition de poudre est dissoute dans un milieu aqueux jusqu'à obtention d'une concentration d'au moins 250 ppm de chlore.

6. Composition à utiliser selon l'une quelconque des revendications 1 à 5, dans laquelle ladite composition de poudre se compose de :
- 25 à 35 % en poids de trichloroisocyanurate de sodium,
- 5 à 15 % en poids de sel de sodium d'EDTA,
- 45 à 60 % en poids de gomme de xanthane,
- 0 à 5 % en poids de carbonate de sodium ou potassium, et
- 2 à 5 % en poids d'un agent de teinture.

7. Composition à utiliser selon la revendication 6, dans laquelle ladite composition de poudre se compose de :
- 27 à 30 % en poids de trichlororisocyanurate de sodium,
- 8 à 10 % en poids de sel de sodium d'EDTA,
- 53 à 57 % en poids de gomme de xanthane,
- 2 à 4 % en poids de carbonate de sodium ou potassium, et
- 2 à 4 % en poids d'agent de teinture.

8. Composition de poudre comprenant :
a) de 10 à 40 % en poids, par rapport au poids total de la composition, d'au moins un composé organique chloré choisi dans le groupe consistant en l'acide isocyanurique chloré ou ses sels, un chlorosulfonamide aromatique ou ses sels, et leurs mélanges,
b) de 3 à 20 % en poids, par rapport au poids total de la composition, de sulfate de cuivre,
c) de 1 à 20 % en poids, par rapport au poids total de la composition, d'EDTA ou de son sel ;
d) de 30 à 75 % en poids, par rapport au poids total de la composition, d'une gomme naturelle,
e) de 0 à 6 % en poids d'un carbonate inorganique,
et
des adjuvants et/ou excipients acceptables sur le plan vétérinaire.

9. Composition selon la revendication 8, dans laquelle ledit composé organique chloré est choisi dans le groupe consistant en le trichloroisocyanurate de sodium, le dichloroisocyanurate de sodium, la chloramine T et leurs mélanges.

10. Composition selon la revendication 8 ou 9, comprenant de 25 à 40 % en poids dudit au moins un composé organique chloré.

11. Composition selon l'une quelconque des revendications 8 à 10, comprenant de 4 à 15 % en poids dudit EDTA ou de son sel, de préférence de 4 à 9 % en poids.

12. Composition selon l'une quelconque des revendications 8 à 11, comprenant de 35 à 70 % en poids de ladite gomme naturelle, de préférence de 40 à 60 % en poids.

13. Composition selon l'une quelconque des revendications 8 à 12, dans laquelle ladite gomme naturelle est choisie dans le groupe consistant en la gomme arabique, la gomme adragante, la gomme de guar et la gomme de xanthane.

14. Composition selon l'une quelconque des revendications 8 à 13, dans laquelle ledit carbonate inorganique est le carbonate d'ammonium, le carbonate de baryum, le carbonate de cadmium, le carbonate de calcium, le carbonate de césium, le carbonate de lithium, le carbonate de magnésium, le carbonate de manganèse, le carbonate de plomb, le carbonate de potassium, le carbonate de sodium, le carbonate de strontium et leurs mélanges.

15. Composition selon l'une quelconque des revendications 8 à 14, comprenant en outre un agent de teinture de poudre.
